# EUROPÄISCHE PATENTSCHRIFT

(11) **EP 2 352 018 B1**
(45) Veröffentlichungstag und Bekanntmachung des Hinweises auf die Patenterteilung: **09.01.2013**
(21) Anmeldenummer: 11000173.2
(22) Anmeldetag: 12.10.2009
(51) Int. Cl.: G01N 27/22, G01N 33/36, G01R 27/26

(54) **Vorrichtung zur Bestimmung einer dielektrischen Eigenschaft einer Kondensatoranordnung**
Device for determining a dielectric property of a capacitor arrangement
Dispositif de détermination d'une propriété diélectrique d'un ensemble condensateur

(30) Priorität: 16.10.2008 CH 16462008
(43) Veröffentlichungstag der Anmeldung: 03.08.2011
(62) Teilanmeldung aus: 09748935.5
(73) Patentinhaber: Uster Technologies AG, 8610 Uster (CH)
(72) Erfinder: Gehrig, Reto, 8406 Winterthur (CH)

(56) Entgegenhaltungen:
- EP-A1- 0 924 513
- WO-A2-2004/083813
- DE-A1- 19 535 177
- GB-A- 1 061 635
- US-A- 3 684 954
- US-A- 3 757 211
- US-A- 4 706 203
- US-A- 4 772 844
- US-A1- 2001 008 478
- Helmut Lindner et al: "Taschenbuch der Elektrotechnik und Elektronik", 2004, Carl Hanser Verlag, Leipzig, XP002624463, ISBN: 3-446-22546-3 * Seiten 52-53; Abbildung 2.4 *

## Beschreibung

### FACHGEBIET

Die vorliegende Erfindung liegt auf dem Gebiet der elektrischen Messvorrichtungen. Sie betrifft eine Vorrichtung zur Bestimmung einer dielektrischen Eigenschaft einer Kondensatoranordnung, gemäss dem Oberbegriff des unabhängigen Patentanspruchs. Die Erfindung erlaubt ein automatisches Abgleichen der Vorrichtung.

Ein bevorzugtes Einsatzgebiet für die Erfindung ist die kapazitive Prüfung von länglichen, vorzugsweise textilen Gebilden wie Kardenband, Vorgarn, Garn oder Gewebe. Eine derartige Prüfung kann bspw. die Detektion von Fremdstoffen oder das Erkennen von Änderungen der Masse pro Längeneinheit und/oder die Messung von Feuchtigkeit im Prüfgut zum Ziel haben. Die Erfindung kann bspw. im Produktionsprozess (online) in Garnreinigern auf Spinn- oder Spulmaschinen oder in der Laborprüfung (offline) in Garnprüfgeräten eingesetzt werden.

### STAND DER TECHNIK

Es ist eine Vielzahl verschiedenartiger Vorrichtungen zur Untersuchung oder Prüfung von länglichem textilem Prüfgut wie bspw. Kardenband, Vorgarn, Garn oder Gewebe bekannt. Sie lassen sich nach ihrer Anwendung in die beiden Klassen Laborprüfung (offline) und Prüfung während des Produktionsprozesses (online) einteilen. Die Vorrichtungen verwenden verschiedene bekannte Sensorprinzipien; hier interessiert besonders das kapazitive Messprinzip. Bei diesem ist ein Messkondensator typischerweise als ebener Plattenkondensator ausgeführt und weist eine Durchgangsöffnung für das Prüfgut auf. Der Messkondensator ist Teil eines LC-Oszillators, so dass bei Anregung des LC-Oszillators eine elektrische Wechselspannung am Messkondensator anliegt. Die Durchgangsöffnung wird dadurch mit einem elektrischen Wechselfeld beaufschlagt. Das Prüfgut wird durch den Plattenkondensator hindurch bewegt und dem Wechselfeld ausgesetzt. Es wird ein elektrisches Ausgangssignal des Plattenkondensators detektiert. In einer Auswerteschaltung werden aus dem Ausgangssignal dielektrische Eigenschaften des Prüfgutes ermittelt. Aus den dielektrischen Eigenschaften werden Parameter des Prüfgutes wie Masse pro Längeneinheit und/oder Materialzusammensetzung bestimmt. Ein kapazitiver Garn- oder Bandsensor ist z. B. in der GB-638,365 A beschrieben.

Um genaue, von äusseren Einflüssen wie Lufttemperatur oder Luftfeuchtigkeit nicht beeinflusste Messungen durchführen zu können, wird häufig eine Kompensationsmethode angewendet. Zu diesem Zweck beinhaltet die Vorrichtung nebst dem eigentlichen Messkondensator einen Referenzkondensator. Dieser kann durch Zufügen einer dritten, parallel zu den beiden Messkondensatorplatten angeordneten Kondensatorplatte gebildet werden, wobei die drei Kondensatorplatten zu einer kapazitiven Messschaltung zusammen geschaltet werden. Beispiele für Messschaltungen und geeignete Auswerteschaltungen für deren Ausgangssignale finden sich in den Schriften EP-0'924'513 A1, WO-2006/105676 A1 und WO-2007/115416 A1.

Ohne Prüfgut sollte die Messschaltung bei angelegter Wechselspannung ein Ausgangssignal mit dem Wert Null liefern. In der Praxis genügt es jedoch wegen verschiedener Unvollkommenheiten realer elektrischer Komponenten nicht, die Messschaltung symmetrisch aufzubauen, um ohne Prüfgut ein Nullsignal zu erhalten. Jede einzelne Messschaltung muss zur Symmetrisierung individuell abgeglichen werden. Der Symmetrieabgleich erfolgt in der Produktion durch den Hersteller und bei Bedarf während des Unterhalts durch eine Serviceperson. Zu diesem Zweck wird üblicherweise die Kapazität mindestens eines zur Messkapazität parallel geschalteten Trimmkondensators verändert. Das Trimmen erfolgt manuell mit einem geeigneten Werkzeug, z. B. einem Schraubendreher. Alternativ wird das bekannte Verfahren des Lasertrimmens angewendet. In jedem Fall muss die Vorrichtung für den Abgleich geöffnet werden. Ein solcher manueller Abgleich ist mühsam, zeitaufwändig und kostspielig.

Die DE-10'2005'006'853 A1 offenbart ein Messsystem mit einem Sensorelement, das eine einen ersten Schwingkreis bildende Leiterstruktur aufweist. Ein zweiter Schwingkreis ist mit dem ersten Schwingkreis gekoppelt und beinhaltet ein veränderbares Bauteil, so dass seine Resonanzfrequenz einstellbar ist. Da die beiden Schwingkreise ein gekoppeltes System bilden, lässt sich auch das durch den ersten Schwingkreis bestimmte Resonanzverhalten beeinflussen und damit auf gewünschte Werte hin verschieben. Dadurch können bspw. Abweichungen der kapazitiven oder induktiven Komponente der Leiterstruktur, die ggf. bei der Herstellung oder Montage des Sensorelementes auftreten, kompensiert werden.

Ein kapazitiver Garnreiniger mit einem Messkondensator, durch welchen das Garn durchläuft, ist in der GB-963,258 A gezeigt. Die am Messkondensator liegende Spannung wird abgegriffen und zur Auslösung eines Gamschnittes verwendet. In Serie zum Messkondensator ist ein veränderbarer Widerstand geschaltet, und die Enden der so gebildeten Serieschaltung sind an einen Wechselspannungsgenerator angeschlossen. Die Empfindlichkeit des Garnreinigers kann durch Verändern des Widerstandes eingestellt werden.

Die US-3,768,006 A zeigt eine Vorrichtung und ein Verfahren zur kapazitiven Messung des Wasseranteils in einer Öl-Wasser-Emlulsion, die in einer Rohrleitung fliesst. Dabei bildet die Rohraussenwand die äussere, geerdete Elektrode des Messkondensators, und ein in der Rohrmitte angeordnetes Element die innere Elektrode. Ein Referenzkondensator bekannter Kapazität ist in Serie mit dem Messkondensator verbunden. Ein Wechselspannungsgenerator erzeugt eine Wechselspannung, die an den beiden Kondensatoren angelegt wird. Die Spannung über dem Referenzkondensator wird gemessen und ist ein Mass für den Wasseranteil. Die Spannung über dem mit Messgut gefüllten Messkondensator wird mit einem Regelkreis konstant gehalten, welcher die Ausgangsamplitude des Wechselspannungsgenerators regelt.

Die US-3,684,953 A und die US-3,684,954 A befassen sich mit einer Vorrichtung und einem Verfahren zur quantitativen Bestimmung einer Eigenschaft eines dielektrischen Prüfgutes, insbesondere seines relativen Feuchtegehaltes. Das Prüfgut wird in einen Messkondensator eingebracht. An den Messkondensator wird ein elektrisches Wechselsignal angelegt, das von einem Oszillator mit einem nachgeschalteten Verstärker erzeugt wird. Ein am Messkondensator abgegriffenes Signal wird demoduliert und mit dem am Messkondensator angelegten, ebenfalls demodulierten Wechselsignal verglichen, indem bspw. ein Quotient der beiden Signale gebildet wird. Aus dem Vergleich wird auf den Feuchtegehalt des Prüfgutes geschlossen. Um den Dynamikbereich der Demodulatoren einzuhalten, wird die Verstärkung des Verstärkers mit dem demodulierten Ausgangssignal des Messkondensators geregelt. Für den Nullabgleich ohne Prüfgut ist ein parallel zum Messkondensator geschalteter, veränderlicher Abgleichkondensator vorgesehen.

Aus der US-4,843,879 A ist ein Gerät für die kapazitive Qualitätskontrolle von textilen Fäden bekannt. Es beinhaltet eine Doppelkondensatoranordnung mit einem Messkondensator und einem Referenzkondensator. Die Doppelkondensatoranordnung ist in einem elektrischen Schaltkreis eingebaut. Der Schaltkreis beinhaltet einen Oszillator zum Anlegen zweier Wechselspannungen mit entgegengesetzten Phasen an die beiden äusseren Kondensatorelektroden der Doppelkondensatoranordnung. In jedem Ast zwischen dem Oszillator und der jeweiligen Elektrode befinden sich ein Signalverstärker und ein Abgleichkondensator. Die Abgleichkondensatoren dienen dem Abgleich des Ausgangssignals der Doppelkondensatoranordnung ohne Prüfgut auf den Wert Null.

### DARSTELLUNG DER ERFINDUNG

Es ist deshalb eine Aufgabe der vorliegenden Erfindung, eine Vorrichtung zur Bestimmung mindestens einer dielektrischen Eigenschaft einer Kondensatoranordnung anzugeben, welche die obigen Nachteile nicht aufweist Die Vorrichtung soll einfach, schnell, kostengünstig und insbesondere automatisch abgleichbar sein.

Diese und andere Aufgaben werden durch die erfindungsgemässe Vorrichtung, wie sie im unabhängigen Patentanspruch definiert ist, gelöst. Vorteilhafte Ausführungsformen sind in den abhängigen Patentansprüchen angegeben.

Die Erfindung beruht auf der Idee, Abgleichmittel in einem elektrischen Pfad zwischen einem Wechselsignalgenerator und einer die Kondensatoranordnung beinhaltenden Messschaltung vorzusehen, die mittels eines elektrischen Steuersignals steuerbar sind, um ein Ausgangssignal der Vorrichtung abzugleichen. Mit solchen externen Abgleichmitteln wird die Vorrichtung abgeglichen, ohne in die Kondensatoranordnung selbst einzugreifen. Dies eröffnet die Möglichkeit eines automatischen Abgleichs der Vorrichtung.

Unter dem Begriff ,,Kondensatoranordnung" wird in dieser Schrift eine Anordnung mit zwei Körpern, die vom elektrischen Wechselsignal des Wechselsignalgenerators ungleichartig aufladbar und durch mindestens ein Dielektrikum voneinander getrennt sind, verstanden. In einer bevorzugten Ausführungsform handelt es sich bei der Kondensatoranordnung um einen Kondensator mit zwei voneinander beabstandeten Platten, zwischen denen sich Luft befindet und zwischen die ein zu untersuchendes bewegtes längliches textiles Prüfgut einführbar ist. Unter dem Begriff ,,elektrisches Wechselsignal" wird in dieser Schrift ein elektrisches Spannungs- oder Stromsignal mit mindestens einem sich zeitlich ändernden, periodischen Anteil (AC-Anteil) verstanden, dem zusätzlich ein zeitlich im Wesentlichen konstanter Anteil (DC-Anteil, Offset) überlagert sein kann.

Die erfindungsgemässe Vorrichtung zur Bestimmung mindestens einer dielektrischen Eigenschaft einer Kondensatoranordnung beinhaltet mindestens einen Wechselsignalgenerator zum Anlegen eines elektrischen Wechselsignals an die Kondensatoranordnung. Sie beinhaltet ferner eine Auswerteschaltung zur Auswertung mindestens einer elektrischen Messgrösse eines an der Kondensatoranordnung abgegriffenen elektrischen Signals. Ausserdem beinhaltet die Vorrichtung Abgleichmittel, die in einem elektrischen Pfad zwischen dem mindestens einen Wechselsignalgenerator und der Kondensatoranordnung angeordnet sind und mittels derer mindestens ein Parameter des elektrischen Wechselsignals derart veränderbar ist, dass ein Ausgangssignal der Auswerteschaltung bei definierten, konstanten Bedingungen einen bestimmten Wert, vorzugsweise Null, annimmt. Es sind Steuermittel zur Abgabe eines elektrischen Steuersignals an die Abgleichmittel vorhanden, mittels dessen die Veränderung des mindestens einen Parameters steuerbar ist.

In einer bevorzugten Ausführungsform weist die Vorrichtung eine Rückkopplung auf, mittels deren ein Ausgangssignal der Kondensatoranordnung oder der Auswerteschaltung auf die Steuermittel einwirkt. Die Kondensatoranordnung ist vorzugsweise vom Wechselsignalgenerator derart abgekoppelt, dass sie die Grundfrequenz und die Signalform des angelegten Wechselsignals nicht wesentlich beeinflusst.

Zur Realisierung der Abgleichmittel werden verschiedene beispielhafte Varianten vorgeschlagen, wobei die Aufzählung nicht abschliessend ist:
- Die Abgleichmittel beinhalten eine Mehrzahl von elektrischen Widerständen, die einzeln oder gruppenweise zu- oder wegschaltbar sind.
- Die Abgleichmittel beinhalten einen Modulator für eine Amplitudenmodulation des elektrischen Wechselsignals.
- Die Abgleichmittel beinhalten einen Verstärker mit variabler oder programmierbarer Verstärkung zur Verstärkung des elektrischen Wechselsignals.
- Die Abgleichmittel beinhalten ein digitales Potenziometer oder einen Rejustor.
- Die Abgleichmittel beinhalten eine Kapazitätsdiode.

Die erfindungsgemässe Vorrichtung beinhaltet einen Referenzkondensator, welcher in Serie zur Kondensatoranordnung geschaltet ist. Dabei ist der mindestens eine Wechselsignalgenerator dazu eingerichtet, an die Kondensatoranordnung bzw. an den Referenzkondensator zwei elektrische Wechselspannungen mit entgegengesetzten Phasen anzulegen. Die Abgleichmittel sind im elektrischen Pfad zwischen dem Wechselsignalgenerator und der Kondensatoranordnung und/oder im elektrischen Pfad zwischen dem mindestens einen Wechselsignalgenerator und dem Referenzkondensator angeordnet.

Eine bevorzugte Verwendung der erfindungsgemässen Vorrichtung liegt in der kapazitiven Untersuchung eines bewegten länglichen textilen Prüfgutes wie Kardenband, Vorgarn, Garn oder Gewebe, wobei das bewegte Prüfgut die Kondensatoranordnung beeinflusst.

Im Verfahren zur Bestimmung mindestens einer dielektrischen Eigenschaft einer Kondensatoranordnung wird ein elektrisches Wechselsignal von mindestens einem Wechselsignalgenerator erzeugt und an die Kondensatoranordnung angelegt. Mindestens eine elektrische Messgrösse eines an der Kondensatoranordnung abgegriffenen elektrischen Signals wird ausgewertet. Mindestens ein Parameter des elektrischen Wechselsignals in einem elektrischen Pfad zwischen dem mindestens einen Wechselsignalgenerator und der Kondensatoranordnung wird derart verändert, dass ein Ausgangssignal der Auswertung bei definierten, konstanten Bedingungen einen bestimmten Wert, vorzugsweise Null, annimmt. Die Veränderung des mindestens einen Parameters wird mit einem elektrischen Steuersignal gesteuert.

In einer ersten bevorzugten Ausführungsform des Verfahrens wird die Kondensatoranordnung im Wesentlichen zeitlich unverändert belassen, ein elektrisches Wechselsignal wird an die Kondensatoranordnung angelegt, ein elektrisches Ausgangssignal der Kondensatoranordnung wird abgegriffen, und das elektrische Steuersignal wird durch das Ausgangssignal beeinflusst. Dabei kann die Beeinflussung des elektrischen Steuersignals durch das Ausgangssignal automatisch in einem geschlossenen Regelkreis erfolgen. Diese erste Ausführungsform des Verfahrens kann zum Abgleichen der erfindungsgemässen Vorrichtung angewendet werden, derart, dass das Ausgangssignal bei definierten, konstanten Bedingungen einen bestimmten Wert, vorzugsweise Null, annimmt.

In einer zweiten bevorzugten Ausführungsform des Verfahrens wird die Kondensatoranordnung im Wesentlichen zeitlich unverändert belassen, und das elektrische Steuersignal ist von einem Ausgangssignal der Kondensatoranordnung unabhängig. Dabei kann das elektrische Steuersignal ein sich zeitlich schnell änderndes, synthetisch erzeugtes und/oder vorgängig gespeichertes Signal sein. Diese zweite Ausführungsform des Verfahrens kann zum Simulieren von Messungen mit der erfindungsgemässen Vorrichtung, zum Testen der erfindungsgemässen Vorrichtung oder zum Testen von der erfindungsgemässen Vorrichtung nachgeschalteten Komponenten angewendet werden.

Als unmittelbare Vorteile, welche die Erfindung bietet, können unter anderen folgende genannt werden:
- Erstens braucht der Abgleich nicht in der Messschaltung zu erfolgen. Die Messschaltung ist der wohl empfindlichste Teil der kapazitiven Vorrichtung und ist somit äusserst empfindlich auf Störungen und Nichtlinearitäten. Indem sie Abgleichmittel ausserhalb der Messschaltung anordnet, vermeidet die Erfindung derartige Störungen in der Messschaltung.
- Zweitens kann erfindungsgemäss der Abgleich mit einer beliebigen Genauigkeit erfolgen. Es kann beispielsweise ein digitales Potenziometer eingesetzt werden, dessen Widerstand über eine Schnittstelle mit einer beliebig grossen Auflösung gesteuert werden kann.
- Drittens kann mit der Erfindung das Signal/Rauschverhältnis für all jene kapazitiven Sensoren merklich verbessert werden, bei welchen der Abgleich bisher durch einen trimmbaren Kondensator in der Messschaltung erfolgte. Dies deshalb, weil ein trimmbares Element in der Messschaltung dauernd eine Wirkung auf die Messschaltung ausübt.

Die Erfindung ist nützlich zum Abgleichen der Vorrichtung. Dank der Erfindung wird der Abgleich der Vorrichtung einfach, schnell und kostengünstig. Das Gerät muss nicht geöffnet werden, um die Messschaltung abzugleichen. Der Abgleich kann jederzeit erfolgen. Er kann automatisch vom Gerät selbst vorgenommen werden, ohne Eingriff einer Bedienungsperson. Dadurch wird es auch möglich, einen Abgleich zu irgendeinem beliebigen Zeitpunkt durchzuführen. So könnte ein Abgleich vor jeder Messung, vor jeder zehnten Messung, bei wesentlichen Änderungen von Langzeiteigenschaften des Ausgangssignals oder nach einer grösseren Änderung der Umgebungsbedingungen automatisch durchgeführt werden. Dies kann eine Erhöhung der Messgenauigkeit, der Messzuverlässigkeit, der Reproduzierbarkeit und allgemein eine Verbesserung der Messresultate zur Folge haben.

Die Abgleichmittel können aber nicht nur für das Abgleichen der Vorrichtung nützlich sein. Sie können auch zur Einspeisung von beliebigen elektrischen Steuersignalen in die Vorrichtung dienen. Mittels solcher Steuersignale wird die Vorrichtung gezielt verstimmt. Eine gezielte Verstimmung der Vorrichtung kann dieselbe oder eine ähnliche Wirkung haben wie eine Verstimmung durch eine Änderung der auszumessenden Kapazität. Durch Einspeisen von Steuersignalen in die Vorrichtung über die Abgleichmittel können also Messungen simuliert werden. Dabei sollten sich die Kapazitäten der Vorrichtung, mit Ausnahme der Abgleichmittel, nicht ändern. Simulierte Messungen können z. B. zum Testen einer Auswerteschaltung und zur Fehlersuche in der Auswerteschaltung verwendet werden. Sie können einem Abgleich der Auswerteschaltung, z. B. von in der Auswerteschaltung enthaltenen Filtern, dienen. Sie können auch zum Testen und/oder Einstellen der Vorrichtung dienen. Es ist möglich, vorgängig Ausgangssignale realer Messungen mit derselben Vorrichtung oder einem anderen Sensor aufzuzeichnen, in einem Speicher zu speichern und als Eingangssignale über die Abgleichmittel in die Vorrichtung einzuspeisen. Dadurch kann die Vorrichtung mit einer ,,Aufahme und Wiedergabe"-Funktion (,,record and playback") ausgestattet werden.

### AUFZÄHLUNG DER ZEICHNUNGEN

Nachfolgend wird die Erfindung anhand der schematischen Zeichnungen detailliert erläutert.
Figur 1 zeigt ein vereinfachtes Blockdiagramm einer herkömmlichen Vorrichtung.
Figuren 2-9 zeigen Schaltschemata von verschiedenen Ausführungsformen der erfindungsgemässen Vorrichtung.

### AUSFÜHRUNG DER ERFINDUNG

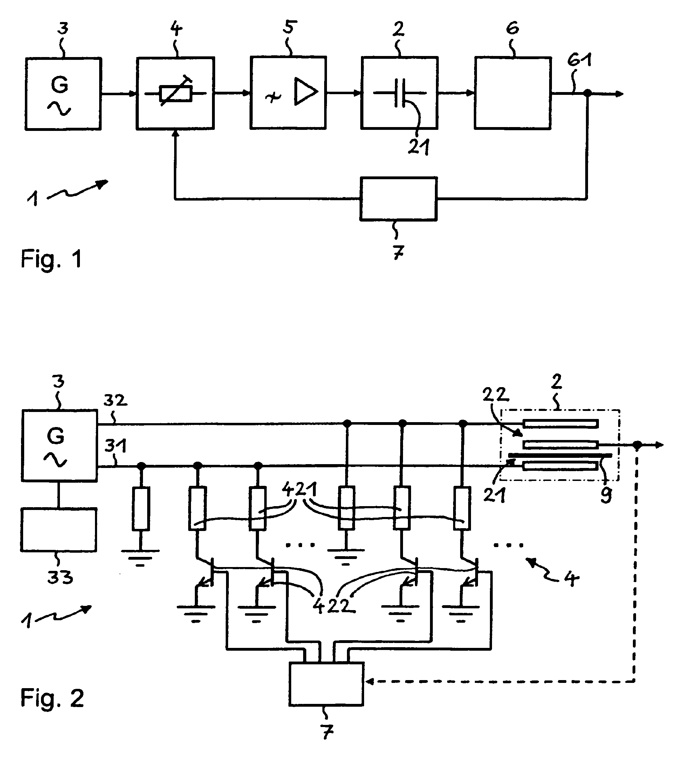

**Figur 1** erklärt anhand eines einfachen Blockdiagramms die Ausgangsbasis der Erfindung. Die Vorrichtung 1 dient zum Ausmessen einer Kapazität 21, welche in einer Messschaltung 2 eingesetzt sein kann. An die Messschaltung 2 wird ein elektrisches Wechselsignal, z. B. eine Wechselspannung, angelegt. Zur Erzeugung des Wechselsignals ist ein Wechselsignalgenerator 3 vorgesehen. Das vom Wechselsignalgenerator 3 erzeugte Wechselsignal kann noch in einer Filter- und/oder Verstärkerstufe 5 gefiltert und/oder verstärkt werden. Die Filter- und/oder Verstärkerstufe 5 dient vorzugsweise auch dazu, die auszumessende Kapazität 21 vom Wechselsignalgenerator 3 derart abzukoppeln, dass sie Parameter des vom Wechselsignalgenerator 3 erzeugten Wechselsignals, z. B. Frequenz, Phase und/oder Amplitude des Wechselsignals, nicht beeinflusst. Der Einfachheit halber ist die Filter- und/oder Verstärkerstufe 5 in den nachfolgenden Figuren nicht mehr eingezeichnet. Der Messschaltung 2 ist vorzugsweise eine Auswerteschaltung 6 nachgeschaltet, die ein Ausgangssignal der Messschaltung 2 auswertet. Die Auswerteschaltung 6 kann als Rechner ausgebildet sein. Sie gibt ihrerseits auf einer Ausgangsleitung 61 ein Ausgangssignal aus, das ein Mass für die auszumessende

Kapazität 21 ist. In der Vorrichtung 1 können selbstverständlich noch weitere Stufen vorgesehen sein. Insbesondere kann es vorteilhaft oder nötig sein, zwischen der Messschaltung 2 und der Auswerteschaltung 6 Filter, Verstärker und/oder Mischer einzubauen, die jedoch in den beiliegenden Zeichnungen der Einfachheit halber nicht dargestellt sind.

In einem elektrischen Pfad zwischen dem Wechselsignalgenerator 3 und der Messschaltung 2 sind Abgleichmittel 4 zum Abgleichen der Vorrichtung 1 angeordnet, mittels derer mindestens ein Parameter, z. B. die Amplitude, des elektrischen Wechselsignals veränderbar ist. Während des Abgleichs der Vorrichtung 1 sollte darauf geachtet werden, dass sich die auszumessende Kapazität 21 und allenfalls andere in der Messschaltung 1 vorhandene Kapazitäten zeitlich nicht ändern. Es sollten also zeitlich konstante Umgebungsbedingungen wie Temperatur und Luftfeuchtigkeit herrschen, oder es sollten entsprechende Änderungen kompensiert werden. Falls die auszumessende Kapazität 21 als Kondensator zur Aufnahme von Prüfgut ausgebildet ist (wie in den nachfolgenden Figuren dargestellt), sollte sich während des Abgleichs kein Prüfgut im Kondensator befinden, oder die Kapazität des Prüfgutes sollte sich zeitlich nicht ändern. Zum Abgleich der Vorrichtung 1 wird ein elektrisches Wechselspannungssignal an die auszumessende Kapazität 21 angelegt. Die Abgleichmittel 4 verändern mindestens einen Parameter des elektrischen Wechselsignals derart, dass das Ausgangssignal der Vorrichtung 1 auf der Ausgangsleitung 61 einen bestimmten Wert annimmt, vorzugsweise null wird. Zu diesem Zweck können die Abgleichmittel 4 manuell von einer Person eingestellt werden, bspw. nach der Herstellung oder beim Unterhalt der Vorrichtung 1. Alternativ können die Abgleichmittel 4 automatisch eingestellt werden. Die automatische Einstellung kann in einer speziell dafür vorgesehenen Steuereinheit 7 oder in der Auswerteeinheit 6 erfolgen. In Figur 1 ist eine Rückkopplung von der Ausgangsleitung 61 zu den Abgleichmitteln 4 eingezeichnet, die ein vom Ausgangssignal der Auswerteeinheit 6 bzw. der Messschaltung 1 abhängiges Steuersignal an die Abgleichmittel 4 abgibt. Mit einer automatischen Einstellung der Abgleichmittel 4 kann die Vorrichtung 1 automatisch abgeglichen werden. Somit liegt ein geschlossener Regelkreis vor, in dem das Ausgangssignal die Regelgrösse ist, die auf den Sollwert Null geregelt werden soll, die Steuereinheit 7 als Regler wirkt und das Steuersignal der Stellwert ist. Alternativ kann die Regelgrösse vor statt hinter der Auswerteeinheit 6 abgegriffen werden.

Eine erste Ausführungsform der erfindungsgemässen Vorrichtung 1 ist in **Figur 2** dargestellt. Die Vorrichtung 1 dient zur kapazitiven Ausmessung eines Prüfgutes 9, z. B. eines Garns. Dazu wird das Prüfgut 9 in einen Messkondensator 21, z. B. einen ebenen Plattenkondensator, eingebracht. Der Messkondensator 21 bildet zusammen mit einem in Serie geschalteten Referenzkondensator 22 und möglicherweise weiteren (nicht eingezeichneten) Komponenten eine kapazitive Messschaltung 2. Der Messschaltung 2 kann, wie in Figur 1, eine Auswerteeinheit 6 nachgeschaltet sein, die jedoch in Figur 2 und in den nachfolgenden Figuren der Übersichtlichkeit halber nicht eingezeichnet ist.

Der Wechselsignalgenerator 3 kann z. B. ein Synthesizer, vorzugsweise ein direkter digitaler Synthesizer (direct digital synthesizer, DDS) sein. Der Synthesizer 3 kann von einer digitalen Schnittstelle 33 angesteuert werden. Der Synthesizer 3 hat vorzugsweise zwei Ausgänge 31, 32 für zwei elektrische Wechselsignale, die im Wesentlichen identisch, aber um 180° gegeneinander phasenverschoben sind. Dem Fachmann sind auch andere Wechselsignalgeneratoren bekannt, die zum Anlegen eines elektrischen Wechselsignals an die Messschaltung 2 geeignet sind, z. B. aus der folgenden Menge: RC-Oszillator, LC-Oszillator, Quarz-Oszillator, Oszillator mit Keramikresonator, Oszillator mit SAW-Komponente (akustische Oberflächenwellen, surface acoustic waves, SAW), Oszillator mit Logikbausteinen, Phasenregelschleife (phase-locked loop, PLL), Pulsweitenmodulator (pulse-width modulator, PWM), Kippstufe.

Im Ausführungsbeispiel von Figur 2 beinhalten die Abgleichmittel 4 eine Mehrzahl von parallel zueinander geschalteten Widerständen 421, die einzeln oder gruppenweise durch Schalter 422, z. B. Hochfrequenztransistoren, zu- und wegschaltbar sind. Dadurch ergibt sich ein veränderbarer Gesamtwiderstand, über welchem eine Spannung entsprechend abfällt, die zudem vom durch den Synthesizer abgegebenen Strom abhängt. Die Widerstände 421 können nur an einem der beiden Synthesizerausgänge oder, wie im Ausführungsbeispiel von Figur 1, an beiden Synthesizerausgängen 31, 32 angebracht sein. Die Schalter 422 können durch eine entsprechende digitale Schnittstelle 7 gesteuert werden.

Zum automatischen Abgleichen der Vorrichtung 1 kann eine Rückkopplung zwischen dem Ausgangssignal der Messschaltung 2 und der digitalen Schnittstelle 7, welche die Schalter 422 ansteuert, vorgesehen sein. Um die Vorrichtung abzugleichen, wird in einer bevorzugten Anwendung kein Prüfgut 9 in den Messkondensator eingeführt, und es wird auf zeitlich möglichst unveränderliche Umgebungsbedingungen wie Luftfeuchtigkeit und Temperatur geachtet. Mittels des Synthesizers 3 wird ein elektrisches Wechselsignal an die Messschaltung 2 angelegt, und die Stellung der Schalter 422 wird durch die digitale Schnittstelle 7 so geregelt, dass das Ausgangssignal der Messschaltung 2 null ist. Wenn dies der Fall ist, dann ist die Vorrichtung 1 abgeglichen, und die Stellung der Schalter 422 wird gespeichert und für die eigentlichen Messungen mit dem Prüfgut 9 beibehalten. Die digitale Schnittstelle 7 entspricht somit der Steuereinheit 7 von Figur 1 und wirkt als Regler in einem geschlossenen Regelkreis.

Die Möglichkeit der Rückkopplung und Regelung besteht in allen Ausführungsformen der erfindungsgemässen Vorrichtung 1, ist aber der Einfachheit halber in den nachfolgenden Figuren nicht mehr eingezeichnet. Aus demselben Grund ist auch in den Figuren 2-9 die fakultative Filter- und/oder Verstärkerstufe 5 (siehe Figur 1) nicht eingezeichnet.

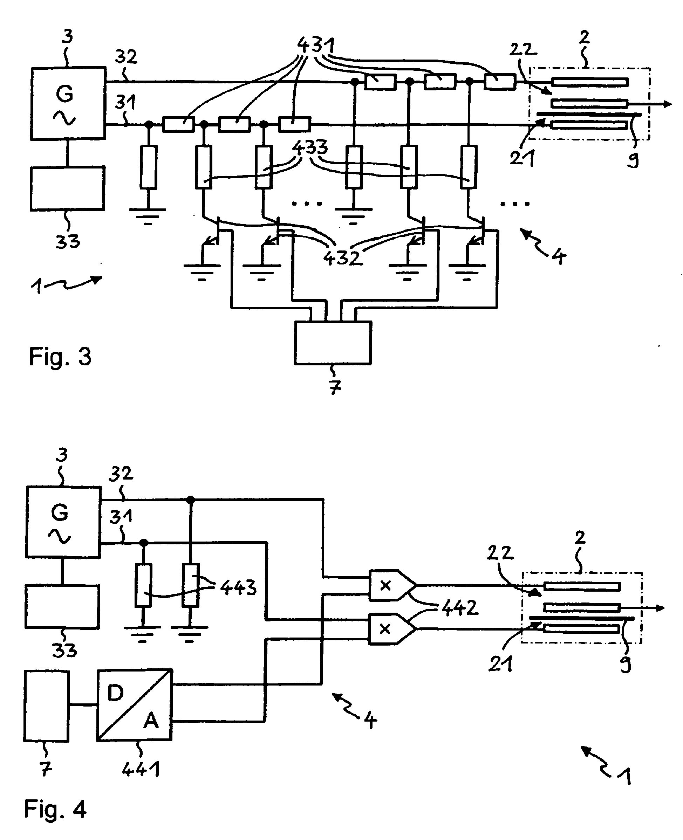

**Figur 3** zeigt eine zweite Ausführungsform der erfindungsgemässen Vorrichtung 1. Hier sind die Abgleichmittel 4 als Widerstandsleitern ausgebildet. Eine solche Widerstandsleiter beinhaltet im Wesentlichen eine Mehrzahl von in Serie geschalteten Widerständen 431, die einzeln oder Gruppenweise durch Schalter 432 über weitere Widerstände 433 zu- oder weggeschaltet werden können. Durch geeignete Schalterstellungen, die wiederum von einer digitalen Schnittstelle 7 gesteuert werden können, kann ein Abgleich der Vorrichtung 1 erreicht werden.

In einer dritten Ausführungsform der erfindungsgemässen Vorrichtung 1 gemäss **Figur 4** kommt ein differenzieller Digital/Analog-Wandler 441 mit zwei Ausgängen beliebiger Bit-Breite zum Einsatz. Der Digital/Analog-Wandler 441 wird von einer geeigneten digitalen Schnittstelle 7 gesteuert. Er erzeugt eine Spannung, die proportional zur Verstimmung bzw. zum Abgleich der Messschaltung 2 ist. Mit dieser Spannung wird in einem analogen Mischer oder Multiplizierer 442 die Wechselspannung des Synthesizers 3 moduliert, und das so modulierte Signal wird an die Messschaltung 2 angelegt. So kann die Amplitude der vom Synthesizer 3 erzeugten Wechselspannung beliebig eingestellt werden, bis die Vorrichtung 1 abgeglichen ist. Die beiden in Figur 4 eingezeichneten Widerstände 443 dienen der Wandlung eines vom Synthesizer 3 gelieferten Wechselstroms in eine Wechselspannung. Sie entfallen, falls der Synthesizer 3 bereits als Wechselspannungsquelle ausgebildet ist. Statt des differenziellen Digital/Analog-Wandlers 441 könnten zwei einpolige Digital/Analog-Wandler eingesetzt werden.

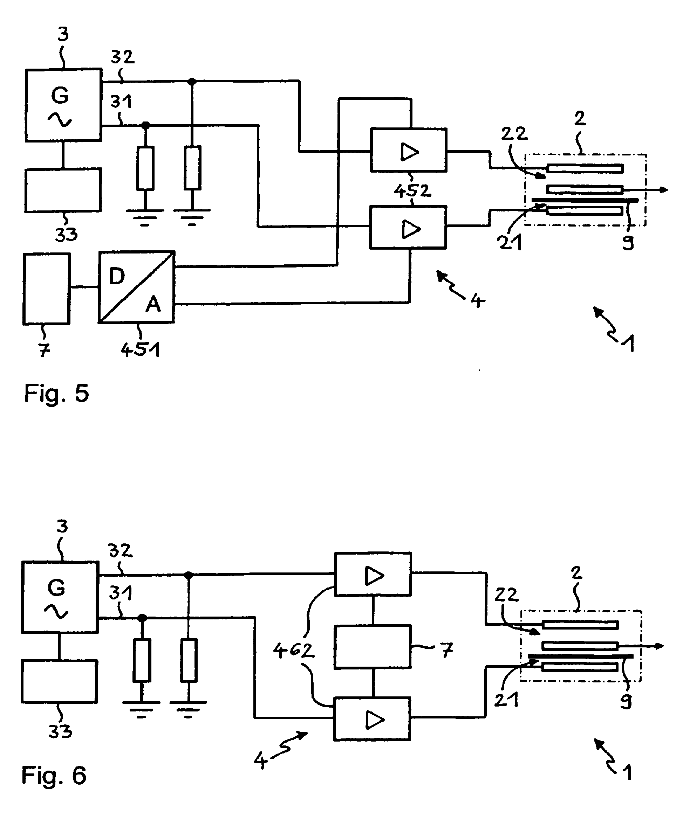

Eine vierte Ausführungsform der erfindungsgemässen Vorrichtung 1, wie sie in **Figur 5** dargestellt ist, funktioniert nach dem Prinzip des spannungsgesteuerten Verstärkers. Sie beinhaltet zwei spannungsgesteuerte variable Verstärker 452 (variable gain amplifier, VGA) für die beiden Ausgangssignale des Synthesizers 3. Die Verstärkungen der variablen Verstärker 452 werden z. B. von einem Digital/Analog-Wandler 451 gesteuert, der seinerseits von einer digitalen Schnittstelle 7 gesteuert wird. Durch Einstellen von geeigneten Verstärkungen für die beiden Ausgangssignale des Synthesizers 3 kann ein Abgleich der Vorrichtung 1 erzielt werden.

Analog zur Ausführungsform von Figur 5 funktioniert eine fünfte Ausführungsform der erfindungsgemässen Vorrichtung 1, die in **Figur 6** gezeigt ist. Statt der analog gesteuerten variablen Verstärker 452 kommen hier jedoch Verstärker 462 mit programmierbarer Verstärkung (programmable gain amplifier, PGA) zum Einsatz. Sie werden von einer geeigneten digitalen Schnittstelle 7 angesteuert. Der Digital/Analog-Wandler 451 entfällt bei dieser Ausführungsform.

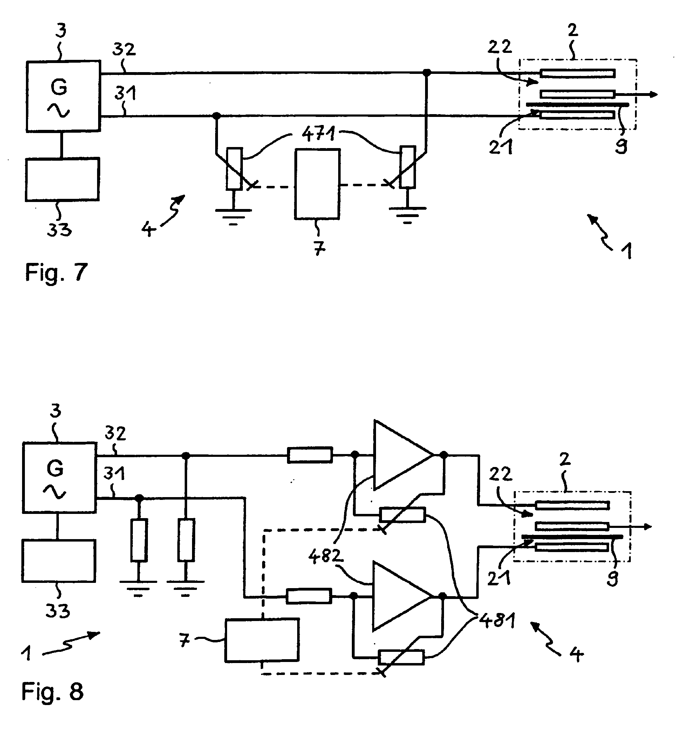

Eine sechste Ausführungsform der erfindungsgemässen Vorrichtung 1 gemäss **Figur 7** verwendet digitale Potenziometer oder Rejustoren 471. Diese können über eine oder zwei digitale Schnittstellen 7 angesteuert werden, wodurch ihr Widerstand so verändert wird, dass ein gezielter Spannungsabfall durch den vom Synthesizer 3 erzeugten Wechselstrom entsteht. Falls der Synthesizer 3 als Wechselspannungsquelle ausgebildet ist, kann die Vorrichtung 1 alternativ als Spannungsteiler beschaltet sein.

Auch in einer siebten Ausführungsform der erfindungsgemässen Vorrichtung 1, die in **Figur 8** dargestellt ist, werden digitale Potenziometer oder Rejustoren 481 eingesetzt, und zwar jeweils bspw. in der Gegenkopplung eines Verstärkers 482, welcher zwischen dem Synthesizer 3 und der Messschaltung 2 ein vom Synthesizer 3 erzeugtes Wechselsignal verstärkt.

Statt der digitalen Potenziometer 481 könnten in den Ausführungsformen der Figuren 7 und 8 alternativ herkömmliche, analoge Potenziometer eingesetzt werden. Mit ihnen könnte eine Bedienungsperson die Vorrichtung 1 manuell abgleichen.

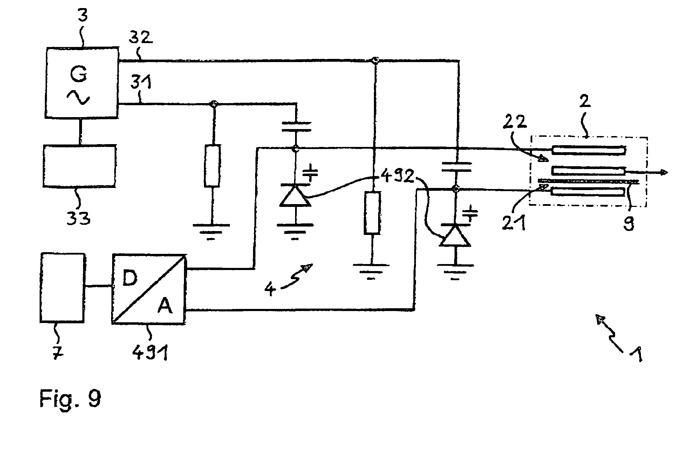

In einer achten Ausführungsform der erfindungsgemässen Vorrichtung 1 gemäss **Figur 9** werden mit Kapazitätsdioden 492 kapazitive Spannungsteiler gebildet. Die Spannungen für die Steuerung der Kapazitätsdioden 492 werden z. B. von einem differenziellen Digital/Analog-Wandler 491 mit zwei Ausgängen geliefert, der seinerseits von einer digitalen Schnittstelle 7 gesteuert wird. Alternativ zum differenziellen Digital/AnalogWandler 491 könnten wiederum zwei einpolige Digital/Analog-Wandler eingesetzt werden.

Selbstverständlich ist die vorliegende Erfindung nicht auf die oben diskutierten Ausführungsformen beschränkt. Bei Kenntnis der Erfindung wird der Fachmann weitere Varianten herleiten können, die auch zum Gegenstand der vorliegenden Erfindung gehören. Solche Varianten können z. B. Kombinationen der oben diskutierten Ausführungsformen sein. Dem Fachmann sind an sich viele elektrische Komponenten bekannt, die als Abgleichmittel 4 für die erfindungsgemässe Vorrichtung 1 in Frage kommen.

### BEZUGSZEICHENLISTE

- 1: Vorrichtung
- 2: Messschaltung
- 21: auszumessende Kapazität, Messkondensator
- 22: Referenzkondensator
- 3: Wechselsignalgenerator
- 31, 32: Ausgänge des Wechselsignalgenerators
- 33: digitale Schnittstelle zur Ansteuerung des Wechselsignalgenerators
- 4: Abgleichmittel
- 411: Digital/Analog-Wandler
- 421: Widerstände
- 422: Schalter
- 431, 433: Widerstände
- 432: Schalter
- 441: Digital/Analog-Wandler
- 442: Multiplizierer
- 451: Digital/Analog-Wandler
- 452: variabler Verstärker
- 462: Verstärker mit variabler Verstärkung
- 471: digitaler Potenziometer oder Rejustor
- 481: digitaler Potenziometer oder Rejustor
- 482: Verstärker
- 491: Digital/Analog-Wandler
- 492: Kapazitätsdiode
- 5: Filter- und/oder Verstärkerstufe
- 6: Auswerteschaltung
- 7: Steuereinheit
- 9: Prüfgut

## Patentansprüche

1. Vorrichtung (1) zur Bestimmung mindestens einer dielektrischen Eigenschaft einer Kondensatoranordnung (21), beinhaltend
eine Auswerteschaltung (6) zur Auswertung mindestens einer elektrischen Messgrösse eines an der Kondensatoranordnung (21) abgegriffenen elektrischen Signals,
einen Referenzkondensator (22), welcher in Serie zur Kondensatoranordnung (21) geschaltet ist, und
mindestens einen Wechselsignalgenerator (3) zum Anlegen von zwei elektrischen Wechselspannungen mit entgegengesetzten Phasen an die Kondensatoranordnung (21) bzw. an den Referenzkondensator (22),
**gekennzeichnet durch**
Abgleichmittel (4), die in einem elektrischen Pfad zwischen dem mindestens einen Wechselsignalgenerator (3) und der Kondensatoranordnung (21) angeordnet sind und mittels derer mindestens ein Parameter des elektrischen Wechselsignals derart veränderbar ist, dass ein Ausgangssignal der Auswerteschaltung (6) bei definierten, konstanten Bedingungen den Wert Null annimmt, und
Steueremittel (7) zur Abgabe eines elektrischen Steuersignals an die Abgleichmittel (4), mittels dessen die Veränderung des mindestens einen Parameters steuerbar ist.

2. Vorrichtung (1) nach Anspruch 1, wobei die Vorrichtung (1) eine Rückkopplung aufweist, mittels deren ein Ausgangssignal der Kondensatoranordnung (21) oder der Auswerteschaltung (6) auf die Steuermittel (7) einwirkt.

3. Vorrichtung (1) nach einem der vorangehenden Ansprüche, wobei die Kondensatoranordnung (21) vom Wechselsignalgenerator (3) derart abgekoppelt ist, dass sie die Grundfrequenz und die Signalform des angelegten Wechselsignals nicht wesentlich beeinflusst.

4. Vorrichtung (1) nach einem der vorangehenden Ansprüche, wobei die Abgleichmittel (4) eine Mehrzahl von elektrischen Widerständen (421, 431, 433) beinhalten, die einzeln oder gruppenweise zu- oder wegschaltbar sind.

5. Vorrichtung (1) nach einem der vorangehenden Ansprüche, wobei die Abgleichmittel (4) einen Modulator (442) für eine Amplitudenmodulation des elektrischen Wechselsignals beinhalten.

6. Vorrichtung (1) nach einem der vorangehenden Ansprüche, wobei die Abgleichmittel (4) einen Verstärker (452, 462) mit variabler oder programmierbarer Verstärkung zur Verstärkung des elektrischen Wechselsignals beinhalten.

7. Vorrichtung (1) nach einem der vorangehenden Ansprüche, wobei die Abgleichmittel (4) ein digitales Potenziometer oder einen Rejustor (471, 481) beinhalten.

8. Vorrichtung (1) nach einem der vorangehenden Ansprüche, wobei die Abgleichmittel (4) eine Kapazitätsdiode (492) beinhalten.

9. Vorrichtung (1) nach einem der vorangehenden Ansprüche, wobei die Abgleichmittel (4) im elektrischen Pfad zwischen dem Wechselsignalgenerator (3) und der Kondensatoranordnung (21) und/oder im elektrischen Pfad zwischen dem mindestens einen Wechselsignalgenerator (3) und dem Referenzkondensator (22) angeordnet sind.

10. Verwendung der Vorrichtung (1) nach einem der vorangehenden Ansprüche zur kapazitiven Untersuchung eines bewegten länglichen textilen Prüfgutes (9) wie Kardenband, Vorgarn, Garn oder Gewebe, wobei das bewegte Prüfgut (9) die Kondensatoranordnung (21) beeinflusst.

## Claims

1. An apparatus (1) for determining at least one dielectric property of a capacitor arrangement (21), containing
an evaluation circuit (6) for evaluating at least one electric measuring quantity of an electric signal tapped from the capacitor arrangement (21),
a reference capacitor (22) which is connected in series to the capacitor arrangement (21), and
at least one alternating signal generator (3) for applying two electric alternating voltages with opposite phases to the capacitor arrangement (21) and the reference capacitor (22), respectively,
**characterized by**
balancing means (4) which are arranged in an electric path between the at least one alternating signal generator (3) and the capacitor arrangement (21) and by means of which at least one parameter of the electric alternating signal is changeable in such a way that an output signal of the evaluation circuit (6) assumes the value zero under defined constant conditions, and
control means (7) for emitting an electric control signal to the balancing means (4), by means of which the change of the at least one parameter is controllable.

2. The apparatus (1) according to claim 1, wherein the apparatus (1) comprises a feedback, by means of which an output signal of the capacitor arrangement (21) or the evaluation circuit (6) acts upon the control means (7).

3. The apparatus (1) according to one of the preceding claims, wherein the capacitor arrangement (21) is decoupled from the alternating signal generator (3) in such a way that it does not relevantly influence the basic frequency and the signal shape of the applied alternating signal.

4. The apparatus (1) according to one of the preceding claims, wherein the balancing means (4) contain a plurality of electric resistors (421, 431, 433) which can be activated and deactivated individually or in groups.

5. The apparatus (1) according to one of the preceding claims, wherein the balancing means (4) contain a modulator (442) for an amplitude modulation of the electric alternating signal.

6. The apparatus (1) according to one of the preceding claims, wherein the balancing means (4) contain an amplifier (452, 462) with variable or programmable gain for amplifying the electric alternating signal.

7. The apparatus (1) according to one of the preceding claims, wherein the balancing means (4) contain a digital potentiometer or a rejustor (471, 481).

8. The apparatus (1) according to one of the preceding claims, wherein the balancing means (4) contain a variable-capacitance diode (492).

9. The apparatus (1) according to claim 11, wherein the balancing means (4) are arranged in the electric path between the alternating signal generator (3) and the capacitor arrangement (21) and/or in the electric path between the at least one alternating signal generator (3) and the reference capacitor (22) .

10. The use of the apparatus (1) according to one of the preceding claims for the capacitive examination of a moved elongated textile test subject (9) such as card sliver, roving yam, yam or fabric, wherein the moved test subject (9) influences the capacitor arrangement (21).

## Revendications

1. Dispositif (1) pour la détermination d'au moins une propriété diélectrique d'une disposition de condensateur (21), comprenant
un circuit d'évaluation (6) destiné à évaluer au moins une grandeur de mesure électrique d'un signal électrique capté sur la disposition de condensateur (21),
un condensateur de référence (22) monté en série avec la disposition de condensateur (21), et
au moins un générateur de signaux alternatifs (3) destiné à appliquer deux courants électriques alternatifs de phase opposée à la disposition de condensateur (21) et au condensateur de référence (22),
**caractérisé en ce qu'**il comporte des moyens d'équilibrage (4) qui sont disposés dans un trajet électrique entre l'au moins un générateur de signaux alternatifs (3) et la disposition de condensateur (21) et au moyen desquels au moins un paramètre du signal électrique alternatif peut être modifié de telle façon qu'un signal de sortie du circuit d'évaluation (6) prenne la valeur zéro dans des conditions constantes définies, et
des moyens de contrôle (7) destinés à émettre un signal électrique de contrôle vers les moyens d'équilibrage (4), au moyen duquel la modification de l'au moins un paramètre est contrôlable.

2. Dispositif (1) selon la revendication 1, lequel dispositif (1) présente un couplage de rétroaction au moyen duquel un signal de sortie de la disposition de condensateur (21) ou du circuit d'évaluation (6) agit sur les moyens de contrôle (7).

3. Dispositif (1) selon l'une des revendications précédentes, dans lequel la disposition de condensateur (21) du générateur de signaux alternatifs (3) est découplée de telle façon qu'elle n'influence pas notablement la fréquence fondamentale ni la forme de signal du signal alternatif appliqué.

4. Dispositif (1) selon l'une des revendications précédentes, dans lequel les moyens d'équilibrage (4) comprennent une pluralité de résistances électriques (421, 431, 433) qui peuvent être mises en circuit ou hors circuit séparément ou par groupes.

5. Dispositif (1) selon l'une des revendications précédentes, dans lequel les moyens d'équilibrage (4) comprennent un modulateur (442) pour une modulation d'amplitude du signal électrique alternatif.

6. Dispositif (1) selon l'une des revendications précédentes, dans lequel les moyens d'équilibrage (4) comprennent un amplificateur (452, 462) à amplification variable ou programmable pour l'amplification du signal électrique alternatif.

7. Dispositif (1) selon l'une des revendications précédentes, dans lequel les moyens d'équilibrage (4) comprennent un potentiomètre numérique ou un rejustor (471, 481).

8. Dispositif (1) selon l'une des revendications précédentes, dans lequel les moyens d'équilibrage (4) comprennent une diode à capacité variable (492).

9. Dispositif (1) selon l'une des revendications précédentes, dans lequel les moyens d'équilibrage (4) sont disposés dans le trajet électrique entre le générateur de signaux alternatifs (3) et la disposition de condensateur (21) et/ou dans le trajet électrique entre l'au moins un générateur de signaux alternatifs (3) et le condensateur de référence (22).

10. Utilisation du dispositif (1) selon l'une des revendications précédentes pour l'analyse capacitive d'une matière textile allongée en mouvement (9) telle qu'un fil de carde, un fil mèche, un filé ou un tissu, dans laquelle la matière textile en mouvement (9) influence la disposition de condensateur (21).
